# EUROPÄISCHE PATENTSCHRIFT

(11) **EP 2 670 293 B1**
(45) Veröffentlichungstag und Bekanntmachung des Hinweises auf die Patenterteilung: **15.07.2020**
(21) Anmeldenummer: 12705612.5
(22) Anmeldetag: 03.02.2012
(51) Int. Cl.: A61B 3/10

(54) **VERFAHREN UND VORRICHTUNG FÜR DIE SEQUENZIELLE AUFNAHME VON INTERFEROMETRISCHEN TIEFENSCHNITTBILDERN IN VERSCHIEDENEN TIEFEN, INSBESONDERE ZUR ANALYSE DES AUGES**
METHOD AND DEVICE FOR THE SEQUENTIAL RECORDING OF INTERFEROMETRIC DEEP SECTIONAL IMAGES AT DIFFERENT DEPTHS, IN PARTICULAR FOR ANALYSIS OF THE EYE
PROCÉDÉ ET DISPOSITIF POUR UNE PRISE DE VUE SÉQUENTIELLE DE VUES EN COUPE PROFONDE INTERFÉROMÉTRIQUES EN DIFFÉRENTES PROFONDEURS, NOTAMMENT POUR L'ANALYSE DE L'OEIL

(30) Priorität: 04.02.2011 DE 102011010443
(43) Veröffentlichungstag der Anmeldung: 11.12.2013
(73) Patentinhaber: Heidelberg Engineering GmbH, 69121 Heidelberg (DE)
(72) Erfinder: ENGELHARDT, Ralf, 23568 Lübeck (DE); DRÖGE, Gerit, 23568 Lübeck (DE); MARTENSEN, Björn, 23562 Lübeck (DE)
(74) Vertreter: Angerhausen, Christoph
(86) Internationale Anmeldenummer: PCT/EP2012/000487
(87) Internationale Veröffentlichungsnummer: WO 2012/104097

(56) Entgegenhaltungen:
- WO-A1-2010/074098
- US-A1- 2007 076 217

## Beschreibung

Die Erfindung bezieht sich auf ein Verfahren für die sequentielle Aufnahme von interferometrischen Tiefenschnittbildern gemäß den im Oberbegriff des Anspruchs 1 angegebenen Merkmalen. Ferner bezieht sich die Erfindung auf eine Vorrichtung zur Durchführung des Verfahrens. Ein derartiges Verfahren beziehungsweise eine derartige Vorrichtung sind aus der US 2007/0076217 A1 bekannt.

Aus der WO 2010/1074098 A1 sind ein ähnliches Verfahren und eine ähnliche Vorrichtung bekannt. Im Probenweg ist eine linear bewegbare elektrische Einheit mit einem Spiegel vorhanden. Der auf den Spiegel auftreffende Probenstrahl wird in der gleichen Richtung wie dieser reflektiert.

Bei der Aufnahme von interferometrischen Tiefenschnittbildern tritt das Problem auf, dass die Vorrichtung nur eine begrenzte Messtiefe bei einer gegebenen Referenzarmlänge erlaubt. Bei Fourier Domain Systemen ist die Messtiefe durch die Auflösung des Spektralapparats begrenzt. Typischerweise werden bei schnell arbeitenden Systemen Tiefen bis zu 10mm Abstand von der Referenzebene mit vertretbarem Signal-zu-Rauschabstand detektiert.

Zusätzlich begrenzt oftmals die Tiefenschärfe des verwendeten optischen Systems die Messtiefe bei der Aufnahme von interferometrischen Tiefenschnittbildern. Typischerweise können nur Bereiche in der Größenordnung der Rayleighlänge mit guter lateraler Auflösung und vertretbarem Rauschabstand aufgenommen werden. Eine Erweiterung der Messtiefe auf vielfache Rayleighlängen erfordert oftmals eine Anpassung der Fokuslage.

Die Tiefenauflösung von interferometrischen Tiefenschnittbildern hängt u.a. von der Anpassung der Dispersion in den Armen des verwendeten Interferometers ab. Die Dispersion ist bei herkömmlichen Systemen nur für eine Messtiefe angepasst, so dass eine Erweiterung der Messtiefe in der Regel mit einem Verlust von axialer Auflösung verbunden ist.

Erfindungsgemäß sollen diese hier aufgezählten Probleme für schnell arbeitende interferometrische Aufnahmesysteme gelöst werden.

Um den mit gutem Signal-zu-Rauschabstand abgetasteten Bereich einer Probe auf eine neue Tiefe einzustellen, muss die Weglängendifferenz zwischen Probenweg und Referenzweg sowie gegebenenfalls die Abbildungsgeometrie und die Fokuslage als auch die Dispersion verändert werden. Insbesondere bei beweglichen Proben (z.B. bei der Vermessung des Auges) muss die Veränderung der Weglängendifferenz und falls erforderlich die Veränderung der Fokuslage und gegebenenfalls die Veränderung der Dispersionsanpassung schnell erfolgen, um die Information über die relative Lage der abgetasteten Bereiche zueinander zu erhalten und gegebenenfalls ein Gesamt-Tiefenschnittbild aus mehreren sequenziell aufgenommenen einzelnen Tiefenschnittbildern zusammensetzen zu können. Moderne interferometrische Meßsysteme erreichen Bildraten bis zu etlichen 100Hz. Für die Umschaltung auf verschiedene Bereiche innerhalb von wesentlich weniger als 10ms (bspw. 1 ms für maximal 10% Totzeit bei einer Bildrate von 100Hz) ist eine kontrollierte Veränderung der Position der Messapparatur relativ zur Probe oder der Position des Spiegels im Referenzarm nicht möglich, da hier Wege von mehreren mm zurückzulegen sind.

Der Erfindung liegt die Aufgabe zugrunde, ein Verfahren und eine Vorrichtung vorzuschlagen, mit welchen die optischen und geometrischen Wege in einem Probenarm und / oder Referenzarm eines Interferometers schnell zwischen zwei oder mehr Positionen umgeschaltet werden können. Interferometrische Tiefenschnittbilder in verschiedenen Tiefen sollen im jeweils abgetasteten Bereich mit einem verbesserten Signal-zu-Rauschabstand erzeugt und / oder aufgenommen werden können. Weiterhin soll in einfacher Weise ein Tiefenschnittbild mit einer erweiterten Messtiefe in einfacher Weise erzeugt werden können. Das Verfahren soll problemlos und funktionssicher durchführbar sein und mit einem geringem Aufwand zuverlässige und / oder verbesserte Ergebnisse liefern.

Das Verfahren und die Vorrichtung sollen zusätzlich die Möglichkeit bieten, in einzelnen oder allen Positionen die Fokuslage bzw. die Abbildungsgeometrie und/oder die Dispersionsanpassung auf die jeweilige Position anzupassen.

Die Lösung dieser Aufgabe erfolgt hinsichtlich des Verfahrens gemäß den in den Patentansprüchen 1 und 9 angegebenen Merkmalen.

Die Erfindung beschreibt ein Verfahren und eine Vorrichtung für eine Schalteinheit, um die optischen Wege im Proben- und/oder Referenzarm eines Interferometers sehr schnell zwischen zwei oder mehr Positionen umzuschalten. Vorzugsweise befindet sich die Schalteinheit im Probenarm, da dies die geschlossene Ausführung des Referenzarms z.B. in einem Faserinterferometer erlaubt. Grundsätzlich ist der Schalter aber mit Einschränkungen der Funktionalität in beiden Armen möglich. Durch die Schalteinheit können ebenfalls weitere Parameter wie Position des Fokus, Abbildungsgeometrie und Dispersion durch Einbringen von Elementen in die geschalteten Wege modifiziert werden. Durch das schnelle Umschalten wird die Verlagerung des Messbereichs in verschiedene Tiefen bei minimaler Totzeit ermöglicht. Die Erfindung wird insbesondere mittels eines Michelson-Interferometers realisiert, doch können im Rahmen der Erfindung andere Interferometer wie beispielsweise Mach-Zehnder-Anordnungen vorgesehen sein.

Erfindungsgemäß wird die Weglängenschalteinheit mit drehteren/Spiegeln realisiert. Hierdurch können die erforderlichen schnellen Umschaltzeiten erreicht werden und es besteht die Möglichkeit, die Strahlengänge für die verschiedenen Positionen auf unterschiedliche optische Wege und durch unterschiedliche optische Elemente zu leiten. Ein auf einem Galvanometermotor montierter Spiegel kann nach dem Stand der Technik innerhalb von 0.1 ms auf eine neue Position eingestellt werden.

Besondere Weiterbildungen und Ausgestaltungen sind in den Unteransprüchen sowie der Beschreibung von in der Zeichnung dargestellten Ausführungsbeispielen angegeben. Die Erfindung wird nachfolgend anhand der besonderen in der Zeichnung dargestellten Ausführungsbeispiele näher erläutert, ohne dass insoweit eine Beschränkung erfolgt. Es zeigen in Prinzipdarstellungen:
- Fig. 1 bis 3: eine typische interferometrische Messanordnung,
- Fig. 4, 5: ein Ausführungsbeispiel der erfindungsgemäßen interferometrischen Messanordnung mit einer Weglängenschalteinheit,
- Fig. 6: eine besondere Ausführungsform der Weglängenschalteinheit,
- Fig. 7: die Weglängenschalteinheit entsprechend Fig. 6 mit integrierten optischen Komponenten,
- Fig. 8: das Zusammensetzen von Einzel-Tiefenschnittbildem in verschiedenen Tiefen zu einem Gesamt-Tiefenschnittbild,
- Fig. 9, 10: weitere besondere Ausführungsbeispiele der Weglängenschalteinheit,
- Fig. 11, 12: Vorrichtungen zur Analyse eines Auges.

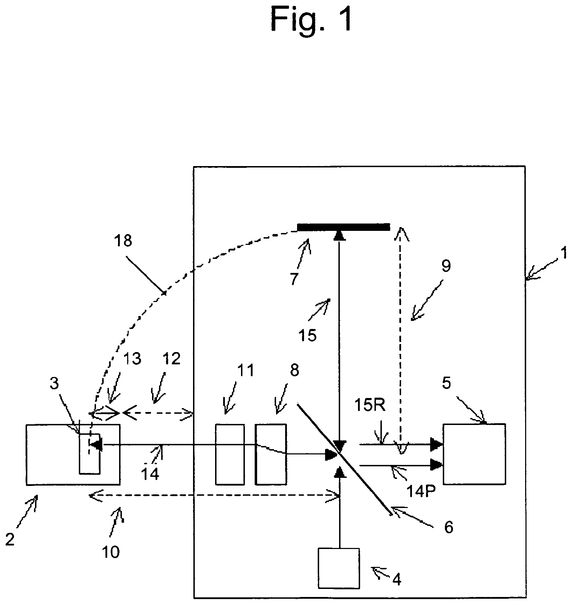

Fig. 1 zeigt eine typische interferometrische Messanordnung zur Aufnahme von Tiefenschnittbildern, hier basierend auf einem Michelson-Interferometer. In einem Bereich 3 innerhalb der Probe 2 wird durch den als Interferometer ausgebildeten Messapparat 1 ein Tiefenschnittbild aufgenommen. Aus der Lichtquelle 4 fällt das Licht auf den Strahlteiler 6 und wird in den Probenstrahl 14 und Referenzstrahl 15 aufgeteilt. Mittels der Pfeile an den jeweiligen Enden des Probenstrahls 14 und des Referenzstrahls 15 sind die Richtungen der von der Lichtquelle 4 ausgesandten Beleuchtungsstrahlen und ferner die reflektierten oder reemittierten entsprechenden Lichtstrahlen angedeutet. Das Tiefenschnittbild wird in der Auswerteeinheit 5 durch Auswertung der interferometrischen Signale berechnet, die sich durch Überlagerung der Probenstrahlen 14P und Referenzstrahlen 15R an verschiedenen lateralen Positionen auf der Probe ergeben. Zur Erzeugung von Tiefenschnittbildern wird der Probenstrahl 14 durch die Ablenkeinheit 8 an verschiedene laterale Positionen der Probe 2 gelenkt. Die optionale optische Einheit 11 fokussiert den Probenstrahl 14 falls erforderlich in einer vorgegebenen Tiefe des Objektes bzw. der Probe und / oder des Messbereichs. Das Tiefenschnittbild wird in der Tiefe 13 in der Probe aufgenommen, an welcher der optische Referenzweg 9 und der optische Probenweg 10 gleich sind, wie mit gestrichelter Linie 18 angedeutet. Die Tiefe wird einerseits durch die Position des Spiegels 7 und andererseits den Abstand 12 der Messapparatur 1 zur Probe 2 bestimmt.

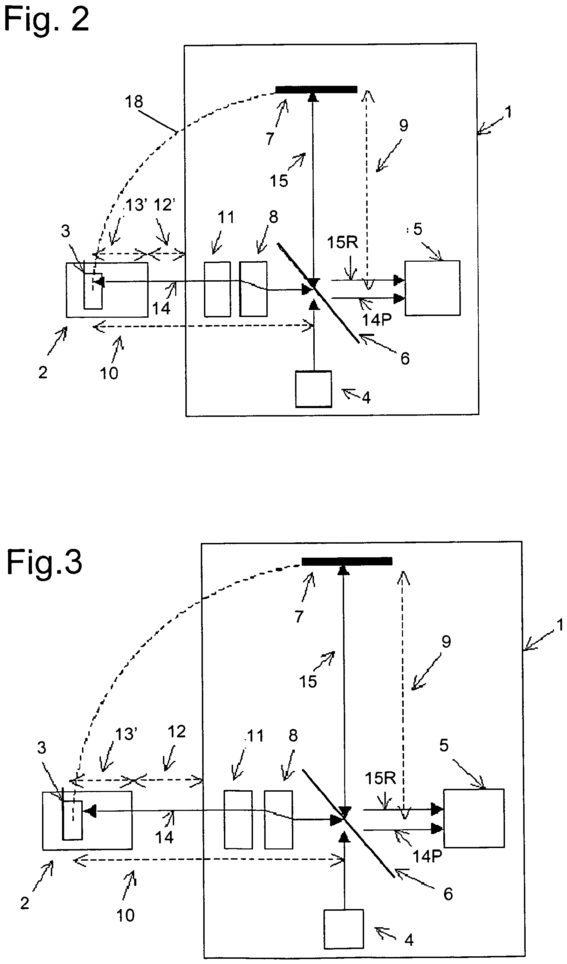

Wenn der Messbereich 3 für das Tiefenschnittbild auf eine neue Tiefe 13 eingestellt werden soll, kann der Abstand zwischen Messapparatur 1 und der Probe 2 verändert werden, wie in Fig. 2 dargestellt. Durch Veränderung des Abstands 12' wird hier der Messbereich in die neue Tiefe 13' gelegt. Das gleiche Ergebnis kann man unter Beibehaltung des Abstands 12 durch Veränderung des optischen Weges 9 des Referenzstrahls 15 erzielen, wie in Fig. 3 dargestellt. Auch hier wird der Messbereich 3 in die neue Tiefe 13' verschoben.

Durch eine sequenzielle Aufnahme von wenigstens zwei Tiefenschnittbildern in verschiedenen Tiefen wird erfindungsgemäß durch Zusammensetzen der einzelnen Bilder ein Gesamt-Tiefenschnittbild mit einer erweiterten Messtiefe erzeugt.

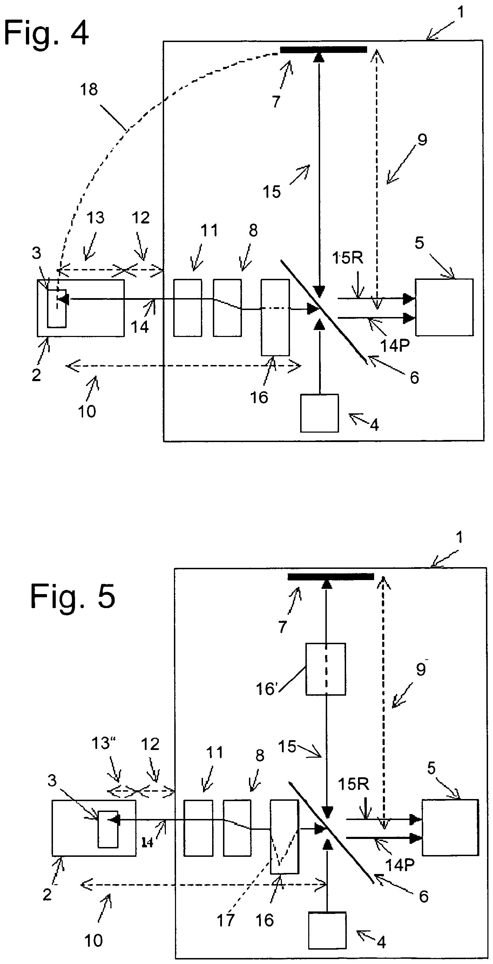

In Fig. 4 ist das Prinzip am Beispiel einer in den Probenweg 14 erfindungsgemäß eingefügten Weglängenschalteinheit 16, wobei die Position des Messbereichs 3 in der Tiefe 13 bei einem kurzen optischen Weg in der Weglängenschalteinheit in der Tiefe 13 innerhalb der Probe 3 liegt. Gemäß Fig. 5 ist die optische Weglänge auf einen längeren optischen Weg in der Weglängenschalteinheit umgeschaltet, wie mittels der gegenüber Fig. 4 verlängerten gestrichelten Linie 17 angedeutet. Wenn alle anderen Größen unverändert sind, wird jetzt ein Messbereich in einer neuen geringeren Tiefe 13" innerhalb der Probe 2 abgetastet. Durch die Ausgestaltung der Weglängenschalteinheit 16 können erfindungsgemäß zwei oder mehr Messbereichspositionen realisiert werden. Der Fokus der optischen Einheit 11 ist erfindungsgemäß angepasst an die mittels der Weglängenschalteinheit 16 eingestellte optische Weglänge und / oder die eingestellte Position des Messbereichs 3.

In Fig. 5 ist eine zusätzliche oder alternative Ausführungsform dargestellt, welche im Referenzweg bzw. Referenzstrahl 15 die Weglängenschalteinheit 16' enthält. Mittels dieser Weglängenschalteinheit 16' kann analog zur Weglängenschalteinheit 16 im Probenweg bzw. Probenstrahl 14 die Weglänge im Referenzweg bzw. Referenzstrahl 15 verändert und / oder eingestellt werden.

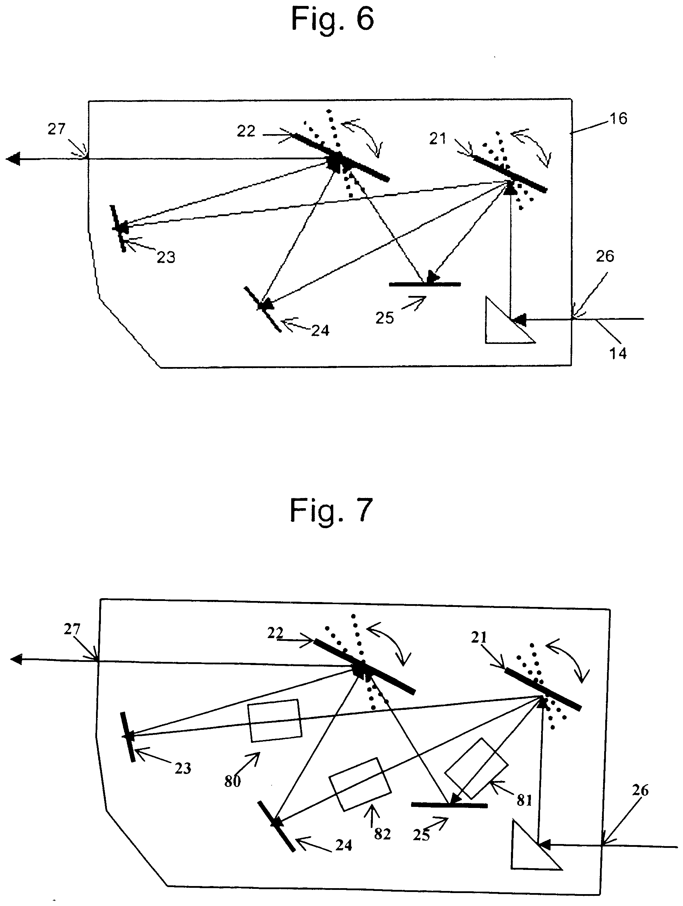

Fig. 6 zeigt eine besondere Ausführungsform der Weglängenschalteinheit 16. Der an der Position 26 einfallende Lichtstrahl 14 wird über den drehbaren Spiegel 21 je nach Spiegelstellung auf einen der Spiegel 23, 24 oder 25 umgelenkt. Die Spiegel 23, 24 und 25 sind jeweils derart justiert, dass der vom Spiegel 21 einfallende Lichtstrahl auf die Achse des drehbaren Spiegels 22 umgelenkt wird. Der Winkel und / oder die Winkelstellung des Spiegels 21 und / oder 22 wird passend und / oder bevorzugt synchron zu den jeweiligen Wegen über den Spiegel 23 oder 24 oder 25 derart eingestellt, dass der Strahl immer an der Position 27 aus der Weglängenschalteinheit 16 austritt. Die Positionen der Spiegel 23, 24 und 25 werden in dieser Ausführungsform so gewählt, dass die Strecke Spiegel 21 - Spiegel 23 - Spiegel 22, die Strecke Spiegel 21 - Spiegel 24 - Spiegel 22, und die Strecke Spiegel 21 - Spiegel 25 - Spiegel 22 voneinander verschieden sind, und zwar um Beträge, der den gewünschten Verschiebungen des Messbereichs entsprechen. Den Spiegeln 21, 22 sind Motoren zugeordnet, welche eine schnelle Einstellung der Spiegel 21, 22 ermöglichen, insbesondere Galvanometer. Mit den drei verschiedenen Kombinationen für die Spiegelwinkel 21 und 22 lassen sich somit drei verschiedene Weglängen zwisehen den Positionen 26 und 27 sehr schnell schalten. Das Prinzip ist nicht auf drei Wege beschränkt, und je nach Ausführungsform der Weglängenschalteinheit 16 können zwei oder mehr Wege realisiert werden. Es versteht sich, dass der reflektierte oder remittierte Lichtstrahl umgekehrt an der Position 27 in die Weglängenschalteinheit 16 eintritt und an der Position 26 austritt.

Je nach Beschaffenheit der Probe und der Messaufgabe kann es erforderlich sein, die Lage des Fokus und/oder die Dispersion der jeweiligen Tiefe des Messbereichs anzupassen. Durch Einbringen von optischen Komponenten in die verschiedenen Wege der Weglängenschalteinheit wird erfindungsgemäß insbesondere unter Berücksichtigung der optischen Einheit 11 gemäß Fig. 1 bis Fig. 5 für jeden der einstellbaren Messbereiche eine unterschiedliche Fokuslage definiert. Außerdem wird durch die Wahl der Dispersion der optischen Komponenten die Dispersion in den verschiedenen Wegen der Weglängenschalteinheit unterschiedlich und / oder angepasst an die Tiefe des Messbereichs eingestellt.

Fig. 7 zeigt eine mögliche Anordnung solcher optischer Komponenten 80, 81 und 82 in der in Fig. 6 dargestellten Anordnung.

In einer weiteren besonderen Ausführungsform ist die Weglängenschalteinheit gemäß Fig. 6 oder Fig. 7 mit der Ablenkeinheit 8 aus Fig. 1 bis Fig. 5 kombiniert, da in der Regel die Ablenkeinheit 8 zur Strahlauslenkung ebenfalls drehbare Spiegel enthält und bevorzugt über den Spiegel 22 in Fig. 6 oder Fig. 7 bereits die Richtung des Probenstrahls verändert werden kann.

Es sei an dieser Stelle darauf hingewiesen, dass die Tiefenschnittbilder in bekannter Weise durch Abrastern oder Scannen in Zeilen und Spalten erfasst und / oder mittels der Auswerteeinheit erzeugt werden. In einer besonderen alternativen Ausgestaltung der Erfindung wird anstelle eines vollständigen Tiefenschnittbilds in einer Tiefe lediglich eine einzige Spalte oder ggf. nur wenige Spalten, insbesondere maximal 10 Spalten, erzeugt. Hierdurch können die Dauer des Verfahrens und / oder der Messzeit nicht unerheblich verkürzt werden.

Durch die Aufnahme von Tiefenschnittbildern an verschiedenen lateralen Positionen können Volumenbilder erzeugt werden. Auch hier kann durch den Einsatz der Weglängenschalteinheit der Messbereich erweitert werden.

Eine große Bedeutung haben interferometrische Messtechniken zur Aufnahme von Tiefenschnittbildern in der medizinischen Diagnostik erhalten. Hier sind diese Messtechniken unter dem Begriff "OCT" (optische Kohärenztomographie) bekannt. Insbesondere in der Ophthalmologie können Tiefenschnittbilder aus großen Tiefen generiert werden und ein großer Messbereich ist erforderlich.

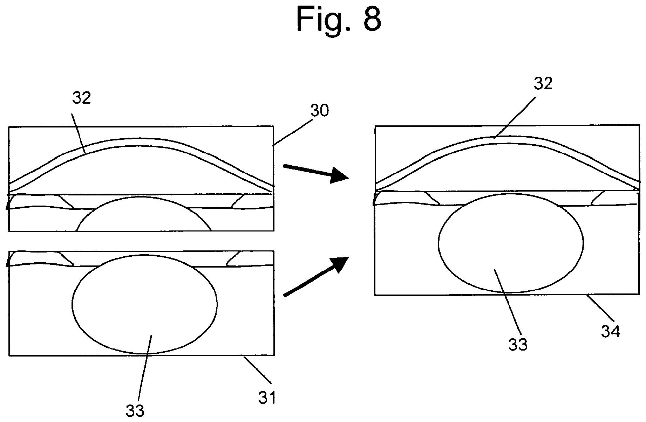

Fig. 8 zeigt schematisch auf der linken Seite mit einem Messbereich von jeweils 8mm aufgenommene Tiefenschnittbilder 30, 31 in unterschiedlichen Tiefen des menschlichen Auges und mit unterschiedlichen Fokuslagen, welche an die unterschiedlichen Tiefen angepasst sind. Diese Aufnahmen wurden mit einer Weglängenschalteinheit mit zwei Wegen aufgenommen. Durch den Einsatz einer Weglängenschalteinheit mit mehr Wegen lassen sich Tiefenschnittbilder über die gesamte Tiefe des Auges erzeugen und zu einem Gesamtbild zusammensetzen. Das obere Tiefenschnittbild 30 zeigt die Cornea 32 und einen Teil der Linse 33, während im unteren Tiefenschnittbild 31 im wesentlichen die Linse 33 zu erkennen ist und von der Cornea 32 lediglich ein kleiner Teilbereich. Wie ersichtlich, weisen die beiden Tiefenschnittbilder 30, 33 einen Überlappungsbereich auf, in welchen gemeinsame Strukturen vorhanden sind. Es ist von Vorteil, wenn die mittels der Weglängenschalteinheit in verschiedenen Tiefen aufgenommenen Tiefenschnittbilder derart vorgegeben werden, dass über einstimmende Überlappungsbereiche vorhanden sind. Aufgrund der im Überlappungsbereich vorhandenen übereinstimmenden Strukturen können erfindungsgemäß Informationen über die relative Lage der einzelnen Tiefenschnittbilder erhalten werden. Aufgrund dieser insbesondere mittels der Auswerteeinheit erhaltenen und / oder erfassten Informationen werden die Tiefenschnittbilder 30, 31 zu einem Gesamt-Tiefenschnittbild 34 zusammengesetzt, wie auf der rechten Seite gezeigt. Dieser Überlappungsbereich ist allerdings nicht zwangsläufig erforderlich, da durch die konstruktive Ausgestaltung der Schalteinheit die relative Lage der Messbereiche festgelegt werden kann und somit bei bekannter Geometrie ein Gesamtbild berechnet werden kann.

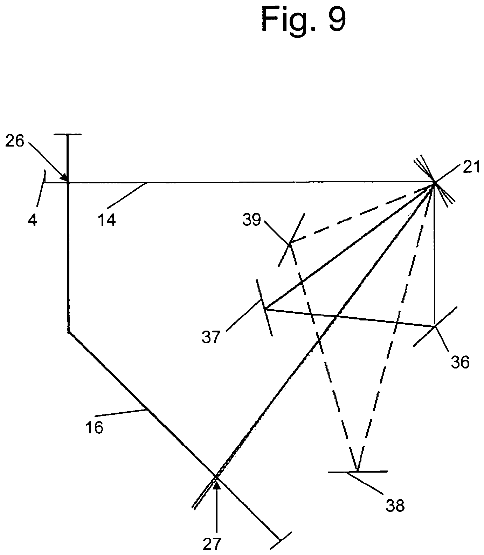

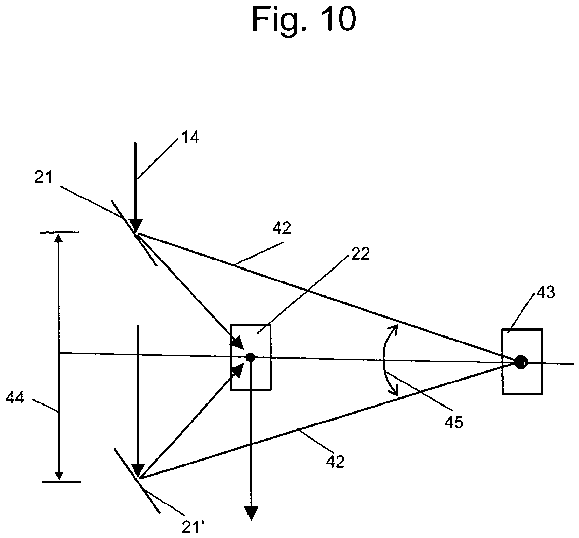

Fig. 9 und 10 zeigen Beispiele für weitere mögliche Ausführungsformen der Weglängenschalteinheit:
Fig. 9 zeigt beispielshaft eine Anordnung mit nur einem verstellbaren Spiegel 21, welche um eine zur Zeichenebene orthogonale Achse drehbar ist und auf welchen der Lichtstrahl 14 der Lichtquelle 4 auftrifft. Es sind zwei einander zugeordnete, in der Weglängenschalteinheit 16 angeordnete, bevorzugt stationäre Spiegelpaare 36, 37 sowie 38, 39 vorhanden. Je nach Winkelstellung des Spiegels 21 gelangt der einfallende Lichtstrahl 14 entweder über das Spiegelpaar 36, 37 oder das Spiegelpaar 38, 39 zurück zum verstellbaren Spiegel 21. Entsprechend der Abstände der Spiegel der jeweiligen Spiegelpaare 36, 37 oder 38, 39 zueinander sowie zum verstellbaren Spiegel 21 sind die jeweiligen Weglängen unterschiedlich.

Es versteht sich, dass im Rahmen der Erfindung mehr als zwei Spiegelpaare oder allgemein Spiegelgruppen vorgesehen werden können. Die Spielgruppen können zudem auch mehr als zwei Spiegel enthalten.

In dem in Fig. 10 gezeigten Ausführungsbeispiel der Weglängenschalteinheit ist der Spiegel 21 über einen Arm 42 mit dem einem Galvanometer 43 verbunden, dessen Schwenkachse orthogonal zur Zeichenebene steht. Mittels des Galvanometers 43 wird der Spiegel 21 zur Weglängenänderung 44 um den Winkel 45 in die dargestellte Position 21' bewegt bzw. geschwenkt. Der weitere Spiegel 22 ist ebenfalls mittels eines hier nicht dargestellten Galvanometers bewegbar bzw. schwenkbar. Die Drehachse bzw. Schwenkachse des weiteren Spiegels 22 weist zur Achse des Galvanometers 43 zweckmäßig einen Abstand auf, welcher im Wesentlichen halb so groß ist wie die Länge des Arms 42. Der Spiegel 22 ist derart angeordnet, dass der vom Spiegel 21 in den beiden dargestellten Positionen reflektierte Lichtstrahl im Wesentlichen auf den Mittelpunkt des weiteren Spiegels 22 gelangt, wobei durch den Mittelpunkt die Dreh- oder Schwenkachse des zugeordneten Galvanometers verläuft. Der konstruktive Aufbau und die Funktionsweise dieser Ausführungsform mit den Spiegeln 21 und 22 samt Galvanometern stimmt mit der Vorrichtung des deutschen Patents DE 41 03 298 C2 überein, dessen Offenbarungsinhalt hiermit ausdrücklich zum Gegenstand der vorliegenden Patentanmeldung erklärt wird.

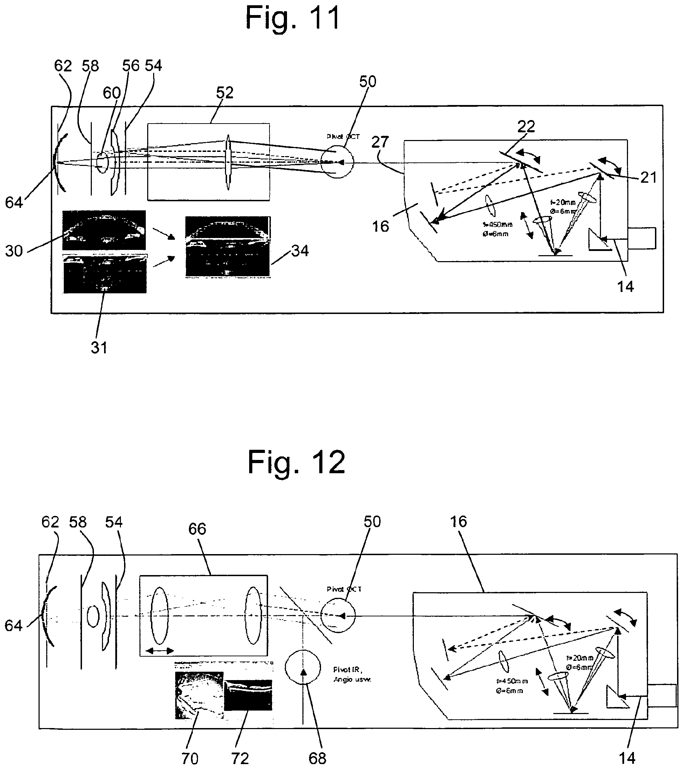

Fig. 11 und 12 zeigen zwei besondere Vorrichtungen zur Durchführung des erfindungsgemäßen Verfahrens, insbesondere zur Analyse des Auges. Durch den Einsatz von Wechselobjektiven oder der Modifizierung der Abbildungsgeometrie durch eine Anordnung von optischen Komponenten im jeweils geschalteten Weg kann mit Hilfe der Weglängenschalteinheit 16 eine Vorrichtung zur Darstellung und Vermessung sämtlicher Strukturen des menschlichen Auges realisiert werden.

Mit einem Kreis 50 ist ein Pivot bzw. eine Systemachse einer Vorrichtung zur optischen Kohärenz Tomographie (OCT) angedeutet. Die optischen Kohärenz Tomographie ermöglicht die Erzeugung von zwei dimensionalen Schnittbildem und / oder Tiefenschnittbildern im Wesentlichen senkrecht zur Netzhautoberfläche, sogenannte B-Scans, und zwar linienförmig aus in die Tiefe gehenden A-Scans. Ferner ist ein Austauschobjektiv 52 für Biometrie und Bildgebung der Vorderkammer vorgesehen. Mit der Linie 54 ist der Fokus des Objektivs 52 im Bereich der Cornea angedeutet, zweckmäßig mit einer Referenz 1 mm vor der Cornea mit Abstand von im Wesentlichen sO + 30 mm. Weiterhin ist mit der Linie 58 der Fokus in der Linse 60 angedeutet, und zwar mit Referenz 1 mm hinter posterior Linse 60, mit im Wesentlichen Abstand sO + 1 mm. Des Weiteren ist mit der Linie 62 der Fokus auf die Retina 64 angedeutet, und zwar mit Referenz vor der Retina sO. Mit der Vorrichtung gemäß Fig. 11 werden zudem die Tiefenschnittbilder 30, 31 erzeugt und zum Gesamt-Tiefenschnittbild 34 zusammengesetzt, wie anhand von Fig. 8 erläutert.

Zur Weglängenschalteinheit 16 wird auf die Erläuterungen der Fig. 6 und 7 Bezug genommen.

Die in Fig. 12 dargestellte Vorrichtung enthält ein Austauschobjektiv 66 für einen Scan der Retina 64. Mit einem Kreis 68 ist ein Pivot und / oder Achse für ein System zur Erzeugung eines Flächenbildes 70 der Retina angedeutet durch Angiografie oder als Reflexionsbild oder Autofluoreszenzbild. Des Weiteren kann mit dieser Vorrichtung das Tiefenschnittbild 72 insbesondere durch Optische Kohärenztomographie (OCT) erzeugt werden. Das System und / oder die Vorrichtung sowie das Verfahren sind in der Patentanmeldung gemäß der Veröffentlichung WO 2008/052 793 A1 beschrieben und anhand der Zeichnung erläutert. Der Offenbarungsinhalt dieser Veröffentlichung wird ausdrücklich zum Bestandteil der vorliegenden Patentanmeldung erklärt.

Die Vorrichtung, insbesondere zur Analyse des Auges, kann auch ohne Wechselobjektive realisiert werden, durch die Umschaltung auf verschiedene Objektive mittels der erfindungsgemäßen Weglängenschalteinheit mit wenigstens einem verstellbaren Spiegel.

### Bezugszeichen

- 1: Messapparat / Interferometer
- 2: Probe
- 3: Bereich in 2 / Messbereich
- 4: Lichtquelle
- 5: Auswerteeinheit
- 6: Strahlteiler
- 7: Spiegel
- 8: Ablenkeinheit
- 9: optischer Referenzweg
- 10: optischer Probenweg
- 11: optische Einheit
- 12: Abstand
- 13: Tiefe
- 14: Probenstrahl / Probenweg
- 14P: Probenstrahl
- 15: Referenzstrahl / Referenzweg
- 15P: Referenzstrahl
- 16: Weglängenschalteinheit
- 17: gestrichelte Linie / Weg in 16
- 18: gestrichelte Linie
- 21, 22: Spiegel bewegbar / drehbar
- 23, 24, 25: Spiegel
- 26, 27: Eintritt- bzw. Austritt Position von 16 des Lichtstrahls
- 30, 31: Tiefenschnittbild
- 32: Cornea
- 33: Linse
- 34: Gesamt-Tiefenschnittbild
- 36-38: stationärer Spiegel
- 42: Arm
- 43: Galvanometer
- 44: Weglängenänderung
- 45: Winkel
- 50: Kreis / Pivot OCT
- 52: Telezentrische Optik
- 54: Linie / Fokus auf 56
- 56: Cornea
- 58: Linie / Fokus auf 60
- 60: Linse
- 62: Linie / Fokus auf 64
- 64: Retina
- 66: Austauschobjektiv
- 68: Kreis / Pivot IR
- 70: Flächenbild
- 72: Tiefenschnittbild

## Patentansprüche

1. Verfahren für die sequenzielle Aufnahme von interferometrischen Schnittbildern in verschiedenen Tiefen, wobei mittels eines Interferometers (1), welches einen optischen Referenzweg (9) und einen optischen Probenweg (10) enthält, ein Probenstrahl (14) einen Messbereich (3) einer Probe (2) abtastet zur Erzeugung eines Tiefenschnittbilds, wobei die Weglänge des Probenstrahls (14) und/oder des Referenzstrahls (15) mittels einer Weglängenschalteinheit (16) verändert wird und Tiefenschnittbilder in wenigstens zwei unterschiedlichen Tiefen der Probe (2) erzeugt werden, wobei mittels wenigstens eines in der Weglängenschalteinheit (16) drehbar angeordneten Spiegels (21, 22), welcher in wenigstens zwei unterschiedliche Winkelpositionen mittels eines Motors eingestellt wird, in der Weglängenschalteinheit (16) die Veränderung der Weglänge durch Umlenken der Strahlengänge für die verschiedenen Winkelpositionen auf unterschiedliche geometrische Wege durch unterschiedliche optische Elemente (23, 24, 25) erfolgt, **dadurch gekennzeichnet, dass**
mittels des wenigstens einen Spiegels (21, 22) der Weglängenschalteinheit (16) der Probenstrahl (14) und/oder der Referenzstrahl (15) derart eingestellt werden, dass unabhängig von der Spiegelstellung der Probenstrahl (14) und/oder der Referenzstrahl (15) an derselben Position (26) der Weglängenschalteinheit (16) eintreten und an derselben Position (27) der Weglängenschalteinheit(16) austreten.

2. Verfahren nach Anspruch 1, **dadurch gekennzeichnet, dass** zur Analyse eines Auges mittels des Probenstrahls (14) ein Messbereich des Auges abgetastet wird.

3. Verfahren nach Anspruch 1 oder 2, **dadurch gekennzeichnet, dass** das Tiefenschnittbild (30, 31) durch Auswertung von interferometrischen Signalen des Interferometers (1), welche sich durch Überlagerung der Probenstrahlen (14) und Referenzstrahlen (15) an verschiedenen lateralen Positionen auf der Probe (2) ergeben, berechnet wird.

4. Verfahren nach einem der Ansprüche 1 bis 3, **dadurch gekennzeichnet, dass** das Tiefenschnittbild (30, 31) durch Auswertung von interferometrischen Signalen des Interferometers (1), welche sich durch Überlagerung der Probenstrahlen (14) und Referenzstrahlen (15) an unterschiedlichen Tiefenpositionen auf der Probe (2) ergeben, berechnet wird.

5. Verfahren nach einem der Ansprüche 1 bis 4, **dadurch gekennzeichnet, dass** mittels einer optischen Einheit (11) der Probenstrahl (14) in eine vorgegebene Tiefe der Probe fokussiert wird, wobei der Fokus auf die mittels der Weglängenschalteinheit (16) vorgegebene Tiefe angepasst ist.

6. Verfahren nach einem der Ansprüche 1 bis 5, **dadurch gekennzeichnet, dass** aus wenigstens zwei Tiefenschnittbildern (30, 31) ein Gesamt-Tiefenschnittbild (34), bevorzugt mittels einer Auswerteeinheit (5), zusammengesetzt wird.

7. Verfahren nach einem der Ansprüche 1 bis 6, **dadurch gekennzeichnet, dass** die relative Lage der abgetasteten Bereiche und / oder Teil-Tiefenschnittbilder (30, 31) erfasst wird.

8. Verfahren nach einem der Ansprüche 1 bis 7, **dadurch gekennzeichnet, dass** unter Berücksichtigung übereinstimmender Referenzsignale der genannten Einzel-Tiefenschnittbilder (30, 31) das Gesamt-Tiefenschnittbild (34), bevorzugt mittels der Auswerteeinheit (5) erzeugt wird, und / oder dass das oder die Referenzsignale aufgrund von gleichen Strukturen, bevorzugt in einem Überlappungsbereich der Einzel-Tiefenschnittbilder (30, 31) erzeugt werden.

9. Vorrichtung für die sequenzielle Aufnahme von interferometrischen Tiefenschnittbildern in verschiedenen Tiefen, insbesondere zur Analyse des Auges, enthaltend ein Interferometer (1) mit einem optischen Referenzweg (9) und einem optischen Probenweg (10), wobei das Interferometer (1) zur Abtastung eines Messbereichs (3) einer Probe (2) mittels eines Probenstrahls (14) ausgebildet ist, zur Durchführung des Verfahrens nach einem der Ansprüche 1 bis 9, wobei im optischen Referenzweg (9) und/oder optischen Probenweg (10) eine Weglängenschalteinheit (16) angeordnet ist, welche wenigstens einen drehbar angeordneten Spiegel (21, 22) enthält und mittels welcher die Länge des optischen Referenzwegs (9) und/oder des optischen Probenwegs (10) derart veränderbar und/oder vorgebbar ist, dass der Messbereich (3) in der Probe (2) in wenigstens zwei voneinander verschiedenen Messtiefen vorgebbar ist, wobei der wenigstens eine drehbar angeordnete Spiegel (21, 22) in wenigstens zwei unterschiedliche Winkelpositionen mittels eines Motors einstellbar ist, **dadurch gekennzeichnet, dass** wenigstens ein weiterer Spiegel (23, 24, 25) derart angeordnet ist, dass in Zusammenwirkung mit dem wenigstens einen drehbar angeordneten Spiegel (21, 22) der Probenstrahl (14) und/oder der Referenzstrahl (15) für beide der zwei unterschiedlichen Winkelpositionen des Spiegels (21, 22) an derselben Position (26) in die Weglängenschalteinheit (16) eintritt und an derselben Position (27) aus der Weglängenschalteinheit (16) austritt.

10. Vorrichtung nach Anspruch 9, **dadurch gekennzeichnet, dass** der drehbare Spiegel (21, 22) in wenigstens zwei verschiedene Positionen mittels eines Motors einstellbar ist, welcher insbesondere als Galvanometer ausgebildet ist.

11. Vorrichtung nach Anspruch 9 oder 10, **dadurch gekennzeichnet, dass** die Weglängenschalteinheit (16) wenigstens einen weiteren Spiegel (23, 24, 25) aufweist, welcher stationär angeordnet ist.

12. Vorrichtung nach einem der Ansprüche 9 bis 11, welche in wenigstens einem der optischen Wege eine optische Einheit (11) aufweist, mit welcher Fokusposition, Dispersion oder Abbildungsgeometrie für den jeweiligen Messbereich vorgebbar ist.

## Claims

1. A method for the sequential recording of interferometric sectional images at different depths, wherein by means of an interferometer (1), which contains an optical reference path (9) and an optical sample path (10), a sample beam (14) scans a measuring region (3) of a sample (2) to produce a depth sectional image, wherein the path length of the sample beam (14) and/or of the reference beam (15) is varied by means of a path length switching unit (16) and depth sectional images are produced at at least two different depths of the sample (2), wherein, by means of at least one mirror (21, 22) which is rotatably arranged in the path length switching unit (16) and which is set in at least two different angular positions by means of a motor, the path length is changed in the path length switching unit (16) by deflecting the beam paths for the different angular positions onto different geometric paths by means of different optical elements (23, 24, 25), **characterised in that**
by means of the at least one mirror (21, 22) of the path length switching unit (16), the sample beam (14) and/or the reference beam (15) can be adjusted in such a way that, irrespective of the mirror position, the sample beam (14) and/or the reference beam (15) enters at the same position (26) of the path length switching unit (16) and exits at the same position (27) of the path length switching unit (16).

2. The method according to claim 1, **characterized in that** for the analysis of an eye by means of the sample beam (14) a measuring area of the eye is scanned.

3. The method according to claim 1 or 2, **characterized in that** the depth section image (30, 31) is calculated by evaluating interferometric signals of the interferometer (1) which result from superimposing the sample beams (14) and reference beams (15) at different lateral positions on the sample (2).

4. The method according to any one of claims 1 to 3, **characterized in that** the depth section image (30, 31) is calculated by evaluating interferometric signals of the interferometer (1) which result from superimposition of the sample beams (14) and reference beams (15) at different depth positions on the sample (2).

5. The method according to one of claims 1 to 4, **characterized in that** by means of an optical unit (11) the sample beam (14) is focused to a predetermined depth of the sample, the focus being adapted to the depth predetermined by means of the path length switching unit (16).

6. The method according to one of claims 1 to 5, **characterized in that** an overall depth sectional image (34) is composed of at least two depth sectional images (30, 31), preferably by means of an evaluation unit (5).

7. The method according to one of claims 1 to 6, **characterized in that** the relative position of the scanned areas and/or partial depth section images (30, 31) is detected.

8. The method according to one of claims 1 to 7, **characterized in that**, taking into account corresponding reference signals of the said individual depth sectional images (30, 31), the overall depth sectional image (34) is generated, preferably by means of the evaluation unit (5), and/or **in that** the reference signal or signals are generated on the basis of identical structures, preferably in an overlapping region of the individual depth sectional images (30, 31).

9. A device for the sequential recording of interferometric depth sectional images at different depths, in particular for analysing the eye, containing an interferometer (1) with an optical reference path (9) and an optical sample path (10), wherein the interferometer (1) is designed to scan a measuring region (3) of a sample (2) by means of a sample beam (14), for carrying out the method according to one of claims 1 to 9, wherein in the optical reference path (9) and/or optical sample path (10) a path length switching unit (16) is arranged, which contains at least one rotatably arranged mirror (21, 22) and by means of which the length of the optical reference path (9) and/or the optical sample path (10) can be changed and/or predetermined in such a way, in that the measuring region (3) in the sample (2) can be preset at at least two different measuring depths, the at least one rotatably arranged mirror (21, 22) being adjustable into at least two different angular positions by means of a motor, **characterized in that** at least one further mirror (23, 24, 25) is arranged in such a way that it can be set at at least two different measuring depths, that in cooperation with the at least one rotatably arranged mirror (21, 22) the sample beam (14) and/or the reference beam (15) for both of the two different angular positions of the mirror (21, 22) enters the path length switching unit (16) at the same position (26) and exits the path length switching unit (16) at the same position (27).

10. The device according to claim 9, **characterized in that** the rotatable mirror (21, 22) can be set in at least two different positions by means of a motor which is designed in particular as a galvanometer.

11. The device according to claim 9 or 10, **characterized in that** the path length switching unit (16) comprises at least one further mirror (23, 24, 25) which is arranged stationary.

12. The device according to one of claims 9 to 11, which has an optical unit (11) in at least one of the optical paths, with which focus position, dispersion or imaging geometry can be specified for the respective measuring range.

## Revendications

1. Procédé pour la prise de vue séquentielle de vues en coupe interférométriques à différentes profondeurs, dans lequel, au moyen d'un interféromètre (1) qui contient un trajet de référence (9) optique et un trajet d'échantillonnage (10) optique, un faisceau d'échantillonnage (14) balaie une zone de mesure (3) d'un échantillon (2) pour la production d'une vue en coupe profonde, dans lequel la longueur de trajet du faisceau d'échantillonnage (14) et/ou du faisceau de référence (15) est modifiée au moyen d'une unité de commutation de longueur de trajet (16), et des vues en coupe profonde sont produites à au moins deux profondeurs différentes de l'échantillon (2), dans lequel, au moyen d'au moins un miroir (21, 22), disposé en rotation dans l'unité de commutation de longueur de trajet (16), lequel est réglé dans au moins deux positions angulaires différentes au moyen d'un moteur, la modification de la longueur de trajet est effectuée dans l'unité de commutation de longueur de trajet (16) par la commutation des trajectoires de faisceau pour les différentes positions angulaires vers différents trajets géométriques par différents éléments optiques (23, 24, 25), **caractérisé en ce que**,
au moyen du miroir (21, 22) au moins au nombre de un de l'unité de commutation de longueur de trajet (16), le faisceau d'échantillonnage (14) et/ou le faisceau de référence (15) sont réglés de telle sorte que, indépendamment du réglage de miroir, le faisceau d'échantillonnage (14) et/ou le faisceau de référence (15) entrent à la même position (26) de l'unité de commutation de longueur de trajet (16) et sortent à la même position (27) de l'unité de commutation de longueur de trajet (16).

2. Procédé selon la revendication 1, **caractérisé en ce qu'**une zone de mesure de l'œil est balayée pour l'analyse d'un œil au moyen du faisceau d'échantillonnage (14).

3. Procédé selon la revendication 1 ou 2, **caractérisé en ce que** la vue en coupe profonde (30, 31) est calculée par l'analyse de signaux interférométriques de l'interféromètre (1), lesquels résultent de la superposition des faisceaux d'échantillonnage (14) et des faisceaux de référence (15) au niveau de différentes positions latérales sur l'échantillon (2).

4. Procédé selon l'une des revendications 1 à 3, **caractérisé en ce que** la vue en coupe profonde (30, 31) est calculée par l'analyse de signaux interférométriques de l'interféromètre (1), lesquels résultent de la superposition des faisceaux d'échantillonnage (14) et des faisceaux de référence (15) au niveau de différentes positions de profondeur sur l'échantillon (2).

5. Procédé selon l'une des revendications 1 à 4, **caractérisé en ce que**, au moyen d'une unité (11) optique, le faisceau d'échantillonnage (14) est concentré à une profondeur prescrite de l'échantillon, dans lequel le foyer est adapté à la profondeur prescrite au moyen de l'unité de commutation de longueur de trajet (16).

6. Procédé selon l'une des revendications 1 à 5, **caractérisé en ce qu'**une vue en coupe profonde globale (34), de préférence au moyen d'une unité d'analyse (5), est composée d'au moins deux vues en coupe profonde (30, 31).

7. Procédé selon l'une des revendications 1 à 6, **caractérisé en ce que** l'emplacement relatif des zones balayées et/ou vues en coupe profonde (30, 31) partielles est détecté.

8. Procédé selon l'une des revendications 1 à 7, **caractérisé en ce que**, en prenant en compte de signaux de référence coïncidents des vues en coupe profonde (30, 31) individuelles citées, la vue en coupe profonde globale (34) est produite de préférence au moyen de l'unité d'analyse (5), et/ou **en ce que** le ou les signaux de référence sont produits sur la base de structures identiques, de préférence dans une zone de superposition des vues en coupe profonde (30, 31) individuelles.

9. Dispositif pour la prise de vue séquentielle de vues en coupe profondes interférométriques à différentes profondeurs, en particulier pour l'analyse de l'œil, contenant un interféromètre (1) avec un trajet de référence (9) optique et un trajet d'échantillonnage (10) optique, l'interféromètre (1) étant constitué pour le balayage d'une zone de mesure (3) d'un échantillon (2) au moyen d'un faisceau d'échantillonnage (14), pour la réalisation du procédé selon l'une des revendications 1 à 9, dans lequel, dans le trajet de référence (9) optique et/ou le trajet d'échantillonnage (10) optique, il est disposé une unité de commutation de longueur de trajet (16) qui contient au moins un miroir (21, 22) disposé en rotation et au moyen de laquelle la longueur du trajet de référence (9) optique et/ou du trajet d'échantillonnage (10) optique peut être modifié et/ou être prescrit de telle sorte que la zone de mesure (3) peut être prescrite dans l'échantillon (2) à au moins deux profondeurs de mesure différentes l'une de l'autre, dans lequel le miroir (21, 22) au moins au nombre de un disposé en rotation peut être réglé dans au moins deux positions angulaires différentes au moyen d'un moteur, **caractérisé en ce qu'**au moins un autre miroir (23, 24, 25) est disposé de telle sorte que, en coopération avec le miroir (21, 22) au moins au nombre de un disposé en rotation, le faisceau d'échantillonnage (14) et/ou le faisceau de référence (15), pour les deux des deux positions angulaires différentes du miroir (21, 22), entre à la même position (26) dans l'unité de commutation de longueur de trajet (16) et sort à la même position (27) de l'unité de commutation de longueur de trajet (16).

10. Dispositif selon la revendication 9, **caractérisé en ce que** le miroir (21, 22) rotatif peut être réglé dans au moins deux positions différentes au moyen d'un moteur, lequel est constitué en particulier en tant que galvanomètre.

11. Dispositif selon la revendication 9 ou 10, **caractérisé en ce que** l'unité de commutation de longueur de trajet (16) comporte au moins un autre miroir (23, 24, 25), lequel est disposé de façon stationnaire.

12. Dispositif selon l'une des revendications 9 à 11, lequel comporte dans au moins l'un des trajets optiques une unité (11) optique avec laquelle la position du foyer, la dispersion ou la géométrie de reproduction peut être prescrite pour la zone de mesure respective.
